# EUROPEAN PATENT APPLICATION

(11) **EP 4 773 258 A1**
(43) Date of publication of application: **08.07.2026**
(21) Application number: 24859836.9
(22) Date of filing: 28.08.2024
(51) Int. Cl.: H01M 4/90, G01N 27/327, G01N 27/416, H01M 4/86, H01M 8/16

(54) **BIOELECTRODE, BIOELECTRODE PRODUCTION METHOD, ELECTROCHEMICAL DEVICE, BLOOD SUGAR LEVEL MEASUREMENT DEVICE, AND CONCENTRATION MEASUREMENT METHOD**

(30) Priority: 30.08.2023 JP 2023140572
(71) Applicant: University of Tsukuba, Ibaraki 305-8577 (JP)
(72) Inventor: TSUJIMURA, Seiya, Tsukuba-shi, Ibaraki 305-8577 (JP); REZKI, Muhammad, Tsukuba-shi, Ibaraki 305-8577 (JP)
(74) Representative: Acapo Onsagers AS
(86) International application number: PCT/JP2024/030737
(87) International publication number: WO 2025/047806

(57) **Abstract**

A bioelectrode includes: a complex compound including a metal and a ligand; a mediator having an anionic functional group; and a conductive material.

## Description

### [Technical Field]

The present disclosure relates to a bioelectrode, a bioelectrode production method, an electrochemical device, a blood sugar level measurement device, and a concentration measurement method.

### [Background Art]

Research and development have been conducted on electrochemical devices, such as a biofuel cell that uses enzymes or microorganisms as a catalyst (hereinafter, also referred to as a biocatalyst) for an electrode and generates power using saccharides, alcohols, or other biomass resources as a fuel.

The electrochemical device utilizing enzymes or microorganisms generally includes a negative electrode (anode) containing a biocatalyst that promotes oxidation of the fuel, and a positive electrode (cathode) containing a biocatalyst that promotes reduction of oxygen. In the negative electrode, electrons that have been extracted by fuel oxidation (for example, a change from glucose to gluconolactone) move to the positive electrode, and oxygen is reduced using the electrons to generate water (H₂O). The movement of electrons caused in this process is used for power generation and the like.

As a case other than a device intended for power generation, for example, a device for measuring a blood sugar level using glucose dehydrogenase, which is an enzyme that catalyzes the oxidation reaction from glucose to gluconolactone, has been put into practical use. In recent years, in addition to a device of a type that measures the blood sugar level of collected blood, a device of a type that continuously measures the blood sugar level of an interstitial fluid over a long period of time by inserting a needle-shaped electrode into a skin is also known.

In the electrochemical device utilizing a biocatalyst, a substance referred to as a mediator that mediates movement of electrons between the biocatalyst and an electrode may be used.

In the electrochemical device using a mediator, it is an important issue as to how to suppress the outflow of the mediator and enhance the durability of an electron transfer function by the mediator.

As a method for enhancing the durability of the electron transfer function by the mediator, various techniques for fixing the mediator to the electrode have been studied.

For example, Non-Patent Document 1 describes a method of immobilizing ferrocene as the mediator on a surface of the electrode by electron beam graft polymerization.

Non-Patent Document 1: J. Power Sources, 479, 228807 (2020)

### [Summary of Invention]

### [Technical Problem]

There is room for improvement in production efficiency in the method described in Non-Patent Document 1 that undergoes processes such as electron beam irradiation and graft polymerization.

In view of the foregoing circumstances, an object of the present disclosure is to provide a bioelectrode, a bioelectrode production method, an electrochemical device, a blood sugar level measurement device, and a concentration measurement method, in which excellent durability of an electron transfer function by a mediator is achieved.

### [Solution to Problem]

Specific means for solving the above-described problems include the following embodiments.
<1> A bioelectrode, including:
   a complex compound composed of a metal and a ligand;
   a mediator having an anionic functional group; and
   a conductive material.
<2> The bioelectrode according to <1>, wherein the metal includes cobalt or zinc.
<3> The bioelectrode according to <1> or <2>, wherein the ligand includes an imidazole compound.
<4> The bioelectrode according to any one of <1> to <3>, wherein the complex compound includes a metal organic framework.
<5> The bioelectrode according to any one of <1> to <4>, further including a biocatalyst.
<6> The bioelectrode according to any one of <1> to <5>, further including chitosan.
<7> An electrochemical device, including the bioelectrode according to any one of <1> to <6>.
<8> A blood sugar level measurement device, including the bioelectrode according to any one of <1> to <6>.
<9> A concentration measurement method, including:
bringing the bioelectrode according to any one of <1> to <6> into contact with a liquid; and
measuring a concentration of a substance contained in the liquid.

### [Effect of Invention]

According to the present disclosure, there are provided a bioelectrode, a bioelectrode production method, an electrochemical device, a blood sugar level measurement device, and a concentration measurement method, in which excellent durability of an electron transfer function by a mediator is achieved.

### [Brief Description of Drawings]

FIG. 1 is a graph of an FTIR result indicating a change in chemical bonding caused by mixing of ZIF67 and 12NQSO.
FIG. 2A is an SEM image of ZIF67-CNT (before mixing with 12NQSO) prepared in Example 1.
FIG. 2B is an SEM image of ZIF67-CNT (after mixing with 12NQSO) prepared in Example 1.
FIG. 3A is an SEM image of ZIF8-CNT (before mixing with 12NQSO) prepared in Example 2.
FIG. 3B is an SEM image of ZIF8-CNT (after mixing with 12NQSO) prepared in Example 2.
FIG. 4 is a graph indicating an electrochemical measurement result of a bioelectrode.
FIG. 5 is a graph indicating the electrochemical measurement result of the bioelectrode.
FIG. 6 is a graph indicating the electrochemical measurement result of the bioelectrode.
FIG. 7 is a graph indicating concentration dependence of sensitivity of the bioelectrode to glucose.
FIG. 8 is a graph indicating selectivity of the sensitivity of the bioelectrode to glucose.
FIG. 9 is a graph indicating durability of the sensitivity of the bioelectrode to glucose.
FIG. 10 is a graph indicating the concentration dependence of the sensitivity of the bioelectrode to lactic acid.
FIG. 11 is a graph indicating the durability of the sensitivity of the bioelectrode to lactic acid.

### [Embodiments for Implementing Invention]

Hereinafter, embodiments for carrying out the present invention will be described in detail. However, the present invention is not limited to the following embodiments. In the following embodiments, the constituent elements (including element steps and the like) are not essential unless otherwise specified. The same applies to numerical values and ranges thereof, and does not limit the present invention.

In the present disclosure, a numerical range that are expressed with "to" includes the numerical values stated before and after "to" as the minimum value and the maximum value, respectively.

### <Bioelectrode>

The bioelectrode of the present disclosure includes a complex compound composed of a metal and a ligand, a mediator having an anionic functional group, and a conductive material.

As indicated in Examples described below, the bioelectrode of the present disclosure exhibits a stable current density.

The reason for this may be, for example, that the mediator contained in the bioelectrode is fixed to the conductive material or the complex compound via an anionic group by a chemical or physical action, whereby a state in which the mediator remains in the bioelectrode is stably maintained.

Furthermore, it is considered that an orientation of an active site of the mediator is controlled by fixing the mediator to the conductive material or the complex compound via the anionic group, whereby electron transfer between a biocatalyst and the electrode is efficiently performed.

In the present disclosure, the term "bioelectrode" refers to an electrode used for an electrochemical device that utilizes a biocatalyst.

When the bioelectrode of the present disclosure is used in the electrochemical device together with the biocatalyst, a favorable electron transfer function between the biocatalyst and the electrode by the mediator is maintained.

### (Complex compound)

The kind of the complex compound contained in the bioelectrode of the present disclosure is not particularly limited.

From the viewpoint of maintaining the state in which the mediator remains in the bioelectrode, the complex compound is preferably a metal organic framework (MOF).

MOF is a complex compound having a crystalline structure formed by crosslinking a metal ion with a ligand. MOF has a large molecular weight and is hardly dissolved in water. Thus, the state in which the mediator remains in the bioelectrode can be effectively maintained together with the conductive material.

### From the viewpoint of thermal stability, chemical stability, and stability in water, the complex compound is more preferably a zeolitic imidazolate framework (ZIF).

ZIF is one of subfamilies of MOF, and is a complex compound formed by crosslinking a metal ion with an imidazole compound as a ligand. ZIF has a cage-like crystalline structure referred to as a sodalite type.

When the complex compound contained in the bioelectrode has a crystalline structure, at least a part of the crystalline structure may be lost.

For example, at least a part of the crystalline structure of the complex compound may be lost when at least a portion of the metal contained in the complex compound forms a coordination bond with an anionic group of a mediator instead of with a ligand.

The type of metal constituting the complex compound is not particularly limited. Specific examples of the metal include divalent metals such as cobalt, zinc, nickel, copper, and iron. From the viewpoint of stability of the complex compound, cobalt and zinc are preferable as the metal.

From the viewpoint of maintaining the state in which the mediator remains in the bioelectrode, it is preferable that the metal contained in the complex compound forms a coordinate bond with an anionic group of the mediator, while appropriately maintaining a coordinate bond with the ligand. From this viewpoint, cobalt is preferable as the metal constituting the complex compound.

The metal constituting the complex compound may be only one kind or two or more kinds.

The type of the ligand constituting the complex compound is not particularly limited. Specific examples of the ligand include imidazole compounds such as 2-methylimidazole, 5-methylimidazole, benzimidazole, 2-imidazole carboxylic acid, 4-imidazole carboxylic acid, 2-nitroimidazole, 4-nitroimidazole, 4,5-dichloroimidazole, 2-ethylimidazole, 5-methylbenzimidazole, 5-chlorobenzimidazole, 5-bromobenzimidazole, and 2-aminobenzimidazole. Among these imidazole compounds, 2-methylimidazole is preferable.

In the present disclosure, the imidazole compound means a compound having an imidazole structure in the molecule.

The ligand constituting the complex compound may be only one kind or two or more kinds.

In an embodiment, the complex compound contained in the bioelectrode is formed from cobalt and 2-methylimidazole. Hereinafter, the complex compound formed from cobalt and 2-methylimidazole is also referred to as ZIF67.

In an embodiment, the complex compound contained in the bioelectrode is formed from zinc and 2-methylimidazole. Hereinafter, the complex compound formed from zinc and 2-methylimidazole is also referred to as ZIF8.

### (Mediator having anionic group)

The mediator contained in the bioelectrode can be selected from compounds having a property of mediating electron transfer between the biocatalyst and the electrode without particular limitation.

Specific examples of the anionic group contained in the mediator include a sulfo group, a carboxy group, and a phospho group. Among these anionic groups, a sulfo group is preferable. The anionic group may form a salt.

In the present disclosure, a state in which the mediator is bonded to a different substance via the anionic group is also included in a case in which "the mediator has an anionic group".

Specific examples of the mediator having an anionic group include naphthoquinone compounds such as 1,2-naphthoquinone-4-sulfonic acid, anthraquinone compounds such as 2-anthraquinone sulfonic acid, and ABTS (2,2'-azino-bis(3-ethylbenzothiazoline-6-sulfonic acid).

The mediator contained in the bioelectrode may be only one kind or two or more kinds.

### (Conductive material)

The material of the conductive material contained in the bioelectrode is not particularly limited, and can be selected from conductive materials such as carbon materials and metals.

Examples of the carbon material include graphite, carbon black (Ketjen black, acetylene black, and the like), MgO template carbon particles (porous carbon particles prepared by a template method), and carbon nanomaterials (carbon nanotube, graphene, carbon nanohorn, carbon nanowire, and the like). The carbon material may be used singly or in combination of two or more kinds thereof.

From the viewpoint of maintaining the state in which the mediator remains in the bioelectrode, the conductive material is preferably in a particulate or fibrous state.

### (Biocatalyst)

The bioelectrode of the present disclosure may include a biocatalyst.

Specific examples of the biocatalyst include enzymes and microorganisms.

When the bioelectrode is used as an anode, the bioelectrode includes a biocatalyst that promotes oxidation of a fuel. The biocatalyst that promotes oxidation of the fuel may be a combination of a biocatalyst that makes the fuel oxidizable by way of hydrolysis or the like, and a biocatalyst that promotes oxidation of the fuel.

The biocatalyst contained in the bioelectrode may be only one kind or two or more kinds.

Examples of the enzyme when the fuel is glucose include glucose oxidase, glucose dehydrogenase, pyranose oxidase, and cellobiose dehydrogenase.

Examples of the enzyme when the fuel is fructose include fructose oxidase and fructose dehydrogenase.

Examples of the enzyme when the fuel is sucrose include a combination of invertase and glucose dehydrogenase.

Examples of the enzyme when the fuel is starch include a combination of amylase and glucose dehydrogenase.

Examples of the enzyme when the fuel is lactic acid include lactate oxidase and lactate dehydrogenase.

Examples of the enzyme when the fuel is alcohol include alcohol oxidase and alcohol dehydrogenase.

Examples of the enzyme when the fuel is aldehyde include aldehyde oxidase and aldehyde dehydrogenase.

Examples of the enzyme when the fuel is pyruvate include pyruvate oxidase and pyruvate dehydrogenase.

When the bioelectrode is used as a cathode, the bioelectrode includes a biocatalyst that promotes reduction of oxygen.

Specific examples of the enzyme that promotes the reduction of oxygen include bilirubin oxidase, laccase, polyphenol oxidase, and ascorbic acid oxidase.

Examples of the microorganism that can be used as the biocatalyst include microorganisms belonging to the genus Shewanella and microorganisms belonging to the genus Geobacter.

### (Other components)

The bioelectrode may include components other than the conductive material, the mediator, and the biocatalyst.

For example, the bioelectrode may include a material for fixing the biocatalyst to the conductive material.

Specific examples of the material for fixing the biocatalyst to the conductive material include a crosslinking agent and a binder. These materials may directly or indirectly fix the biocatalyst to the conductive material.

Examples of the crosslinking agent include compounds having two or more crosslinkable groups in the molecule.

The crosslinking agent binds, for example, molecules of the biocatalyst to each other, molecules of the binder to each other, or the molecule of the biocatalyst to the molecule of the binder to each other.

Examples of the crosslinkable group of the crosslinking agent include a hydroxy group, an epoxy group, an amino group, an aldehyde group, a carboxy group, and an NHS ester group.

Specific examples of the crosslinking agent include genipin, polyethylene glycol diglycidyl ether (PEGDGE), 1,2-bis(2-aminoethoxy)ethane (AEE), and glutaraldehyde.

Examples of the binder include polymer materials such as chitosan (which may be crosslinked with genipin), polyvinylidene fluoride (PVDF), styrene-butadiene rubber (SBR), polytetrafluoroethylene (PTFE), polyvinyl alcohol (PVA), Nafion^{™}, polyethyleneimine, polyallylamine, polystyrene sulfonic acid, carrageenan, and polylysine.

The binder may form an enzyme immobilization film (a film made of a polymer that prevents elution of the enzyme without suppressing permeation of a reactant).

Examples of the polymer that prevents the elution of the enzyme without suppressing the permeation of the reactant include chitosan, Nafion^{™}, polyethyleneimine, polyallylamine, polystyrene sulfonic acid, carrageenan, and polylysine.

### <Bioelectrode production method>

A bioelectrode production method according to the present disclosure includes: preparing a mixture of a complex compound or a precursor of the complex compound and a conductive material; and mixing the mixture with a mediator having an anionic functional group.

According to the above-described method, a bioelectrode having excellent durability of the electron transfer function of the mediator can be produced by a simpler method as compared with other techniques such as electron beam graft polymerization and electropolymerization.

In the above method, the term "precursor of the complex compound" refers to the metal and the ligand used to form the complex compound. The metal may be in the form of a salt.

The process of preparing a mixture of the complex compound or the precursor thereof and the conductive material may be performed simultaneously with the process of mixing the mixture with the mediator having an anionic functional group. That is, the process of preparing a mixture of the complex compound or the precursor thereof and the conductive material may be a process of mixing the complex compound or the precursor thereof, the conductive material, and the mediator having an anionic functional group.

The bioelectrode production method according to the present disclosure may include processes other than the above-described processes as necessary.

For example, the method may include a process of supporting the biocatalyst on the conductive material.

The method for supporting the biocatalyst on the conductive material is not particularly limited, and a known method can be used.

Examples thereof include a method of bringing a liquid in which the biocatalyst is dissolved or dispersed into contact with the conductive material.

The biocatalyst may be fixed to the conductive material by a material such as a crosslinking agent or a binder.

The details and preferred embodiments of each material used in the bioelectrode production method according to the present disclosure are the same as the details and preferred embodiments of each material used in the bioelectrode described above.

### <Electrochemical device>

The electrochemical device according to the present disclosure includes the bioelectrode described above. The electrochemical device may include the bioelectrode described above as an anode, as a cathode, or as both the anode and the cathode.

Specific examples of the electrochemical device include a biofuel cell, a bioreactor, and a biosensor.

Hereinafter, a configuration of a biofuel cell will be described as an example of the electrochemical device.

The biofuel cell includes at least a cathode and an anode.

When the fuel is supplied to the biofuel cell, electrons generated by oxidation of the fuel at the anode move to the cathode and are used for reduction of oxygen at the cathode. The movement of electrons caused in this process is used for power generation.

The electrochemical device according to the present disclosure may include a biocatalyst. Examples of the biocatalyst include the above-described enzymes and microorganisms.

The biocatalyst may be fixed to the electrode or may not be fixed to the electrode (for example, the biocatalyst may be present in an electrolyte).

The type of the fuel used in the biofuel cell is not particularly limited as long as the fuel is a substance whose oxidation is promoted by the biocatalyst. Specific examples of the fuel include saccharides, alcohols, aldehydes, amino acids, amines, organic acids such as acetic acid, lactic acid, and uric acid, and hydrogen.

The fuel used in the biofuel cell may be a single fuel or two or more fuels. The fuel may be one that can be directly oxidized by the biocatalyst in its original form, or one that becomes oxidizable through hydrolysis or the like (for example, starch that is converted into glucose by hydrolysis).

### <Blood sugar level measurement device>

The blood sugar level measurement device according to the present disclosure includes the bioelectrode described above.

The blood sugar level measurement device according to the present disclosure exhibits excellent sensitivity to a wide concentration range of glucose.

The blood sugar level measurement device may contain, as a biocatalyst, an enzyme that uses glucose as a substrate, such as glucose dehydrogenase,

The method for measuring a blood sugar level using the blood sugar level measurement device according to the present disclosure is not particularly limited, and can be selected from known methods.

In an embodiment, the blood sugar level measurement device according to the present disclosure is used for continuous measurement of the blood sugar levels in subcutaneous interstitial fluid. A measurement period of the blood sugar level is not particularly limited, and can be selected, for example, from a range of from 1 hour to 2 weeks.

### <Concentration measurement method>

The concentration measurement method according to the present disclosure includes: bringing the bioelectrode into contact with a liquid; and measuring a concentration of a substance contained in the liquid.

The concentration measurement method according to the present disclosure may measure the concentration of a substance whose oxidation is promoted by the biocatalyst contained in the bioelectrode.

Specific examples of the substance for the concentration measurement include saccharides, alcohols, aldehydes, amino acids, amines, and organic acids such as acetic acid, lactic acid, and uric acid.

The liquid containing the substance for the concentration measurement may be a liquid derived from a living body such as blood or sweat, or may be a liquid not derived from the living body.

The method for measuring the concentration of the substance contained in the liquid that has been brought into contact with the bioelectrode is not particularly limited, and the measurement can be performed by a known method.

### [Examples]

Hereinafter, the present disclosure will be described in more detail based on examples; however, the present disclosure is not limited to these examples.

### <Example 1>

### (1) Preparation of ZIF67-CNT

60 mg of carbon nanotube (CNT) as a conductive material was added to 10 mL of methanol (MtOH) in which 0.24 g of polyvinylpyrrolidone (PVP) was dissolved, and the mixture was stirred for 30 minutes. The recovered CNT was washed with MtOH, added to 5 mL of MtOH, and ultrasonically dispersed for 10 minutes to obtain a CNT dispersion.

0.3 g of Co(NO₃)₂ was dissolved in 2 mL of MtOH to obtain a Co ion solution.

5.765 g of 2-methylimidazole (2-Mim) was dissolved in 20 mL of MtOH to obtain a 2-Mim solution.

The CNT dispersion and the Co ion solution were added to the 2-Mim solution and stirred overnight. Thereafter, a precipitate was washed several times with MtOH and dried at 60°C overnight to obtain powdery ZIF67-CNT.

When ZIF67-CNT was observed with a scanning electron microscope (SEM), a complex compound having a crystalline structure of ZIF67 was formed.

### (2) Mixing of ZIF67-CNT and mediator

To a solution (320 µM) obtained by dissolving 1,2-naphthoquinone-4-sulfonic acid (12NQSO) in 20 mL of water, 20 mg of ZIF67-CNT was added, and the mixture was incubated at room temperature for 24 hours. Subsequently, a precipitate was washed several times with water and dispersed in 1 mL of water to obtain a mixture of ZIF67-CNT and a mediator (ZIF67-CNT/12NQSO).

### (3) Production of bioelectrode

ZIF67-CNT/12NQSO was placed on a surface of a glassy carbon electrode and dried. Subsequently, a mixture of glucose dehydrogenase with FAD as a coenzyme (FAD-GDH), chitosan, and genipin was placed on ZIF67-CNT/12NQSO to obtain a bioelectrode.

The mixture of FAD-GDH, chitosan, and genipin was prepared by mixing a solution (10 µL) containing 1 mass% of chitosan and 1 mass% of acetic acid, a 25 mg/mL FAD-GDH solution (20 µL), and a solution obtained by dissolving 30 mg of genipin in 1 mL of ethanol (10 µL).

### (4) FTIR analysis

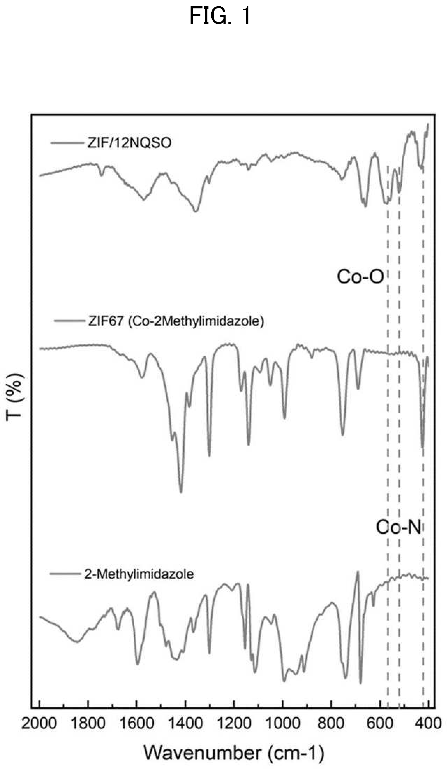

For the purpose of examining a change in chemical bonding due to the mixing of ZIF67 and 12NQSO, FTIR analysis was performed on a mixture of ZIF67 and 12NQSO (ZIF/12NQSO), ZIF67, and 2-methylimidazole. The results are presented in FIG. 1.

As indicated in FIG. 1, a transmittance T (%) of a peak derived from a Co-N bond derived from a coordinate bond between cobalt and a ligand observed in ZIF67 remarkably decreased in ZIF/12NQSO. In addition, a peak derived from a Co-O bond indicating an interaction between Co and an oxygen atom contained in the sulfo group of 12NQSO appeared in ZIF/12NQSO.

The above-described results suggest that when ZIF67 and 12NQSO are mixed, a part of Co constituting ZIF67 forms a coordinate bond with the sulfo group of 12NQSO.

### (5) Electron microscope observation

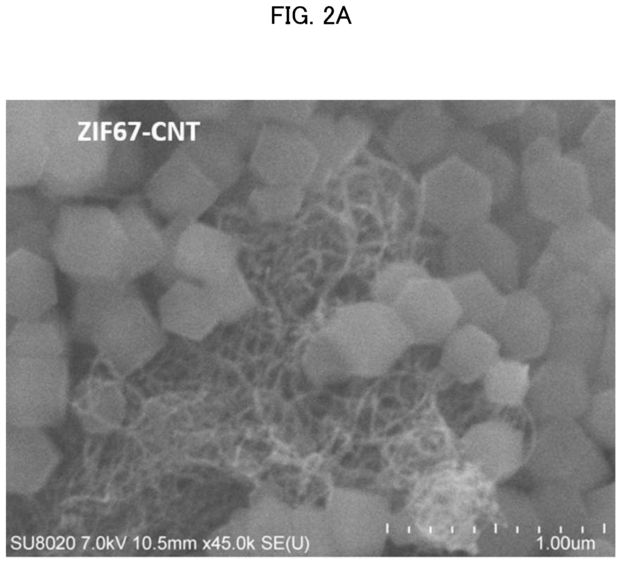

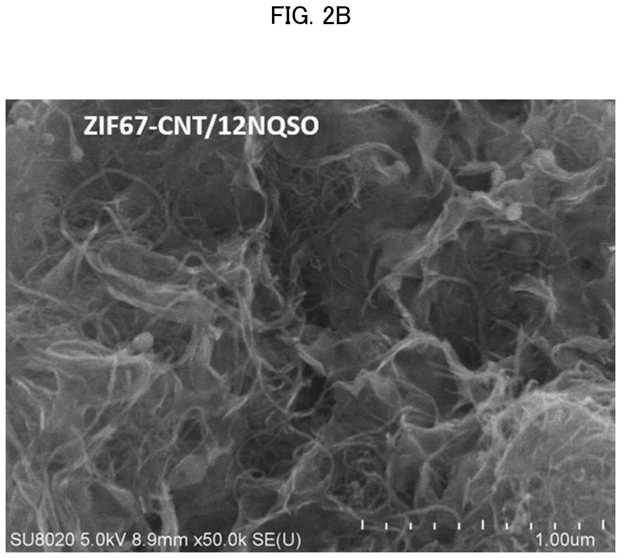

SEM images of ZIF67-CNT prior to mixing with 12NQSO and after mixing with 12NQSO, i.e., ZIF67-CNT/12NQSO, were acquired. The results are indicated in FIGS. 2A and 2B.

As indicated in FIGS. 2A and 2B, the crystalline structure of ZIF67 was confirmed in ZIF67-CNT before being mixed with 12NQSO. On the other hand, the crystalline structure of ZIF67 was not confirmed in ZIF67-CNT/12NQSO after mixing with 12NQSO.

The collapse of the original structure of ZIF-67 is considered to be attributable to partial disruption of the metal-ligand coordination in ZIF-67 and the formation of a new bond between the metal and 12NQSO, as evidenced by the FTIR results shown in Fig. 1.

### <Example 2>

### (1) Preparation of ZIF8-CNT

60 mg of carbon nanotube (CNT) as a conductive material was added to 10 mL of methanol (MtOH) in which 0.24 g of polyvinylpyrrolidone (PVP) was dissolved, and the mixture was stirred for 30 minutes. The recovered CNT was washed with MtOH, and added to 5 mL of MtOH to obtain a CNT dispersion.

0.3 g of Zn(NO₃)₂·6H₂O was dissolved in 2 mL of MtOH to obtain a Zn ion solution.

5.765 g of 2-methylimidazole (2-Mim) was dissolved in 20 mL of MtOH to obtain a 2-Mim solution.

The CNT dispersion and the Zn ion solution were added to the 2-Mim solution and stirred overnight. Thereafter, a precipitate was washed several times with MtOH and dried at 60°C overnight to obtain powdery ZIF8-CNT.

When ZIF8-CNT was observed with a scanning electron microscope (SEM), a complex compound having a crystalline structure of ZIF8 was formed.

### (2) Mixing of ZIF8-CNT and mediator

A mixture of ZIF8-CNT and a mediator (ZIF8-CNT/12NQSO) was obtained in the same manner as in Example 1 except that ZIF8-CNT was used in place of ZIF67-CNT.

### (3) Production of bioelectrode

A bioelectrode was produced in the same manner as in Example 1 except that ZIF8-CNT/12NQSO was used in place of ZIF67-CNT/12NQSO.

### (4) Electron microscope observation

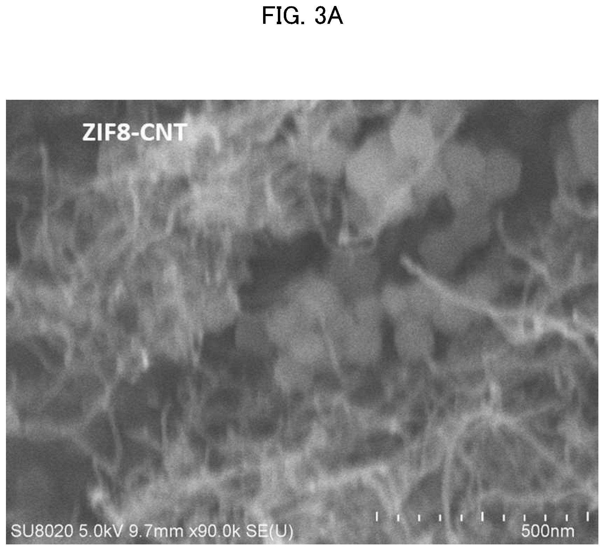

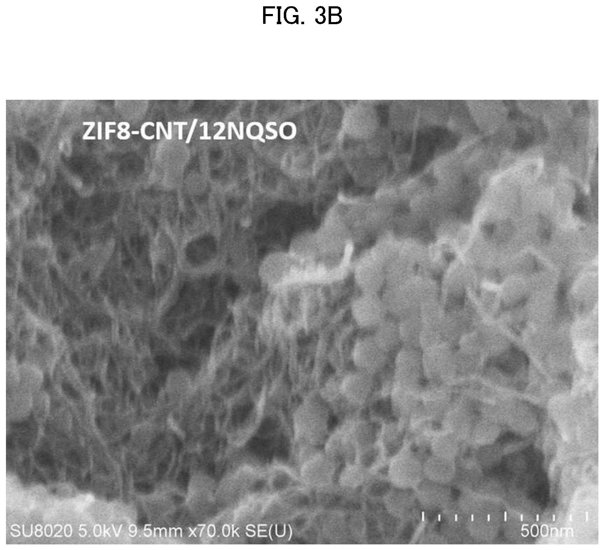

SEM images of ZIF8-CNT prior to mixing with 12NQSO and after mixing with 12NQSO, i.e., ZIF8-CNT/12NQSO, were acquired. The results are presented in FIGS. 3A and 3B.

As indicated in FIGS. 3A and 3B, the crystalline structure of ZIF8 was confirmed in ZIF8-CNT before being mixed with 12NQSO. Unlike Example 1, the crystalline structure of ZIF8 was also confirmed in ZIF8-CNT/12NQSO after mixing with 12NQSO.

### <Comparative Example 1>

A bioelectrode was produced in the same manner as in Example 1 except that CNT not being mixed with the Co ion solution and the 2-Mim solution was used in place of ZIF67-CNT.

### <Comparative Example 2>

A bioelectrode was produced in the same manner as in Example 1 except that ZIF67 obtained by mixing the Co ion solution and the 2-Mim solution was used in place of ZIF67-CNT.

### <Comparative Example 3>

A bioelectrode was produced in the same manner as in Example 1 except that 12NQSO (5 mM) was used in place of ZIF67-CNT.

### <Electrochemical measurement>

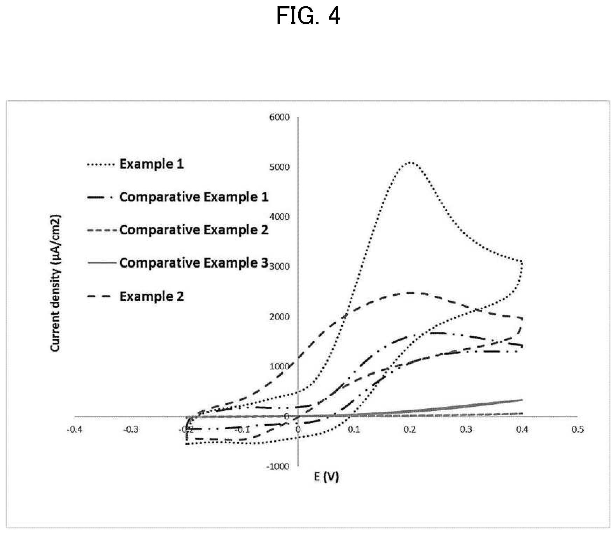

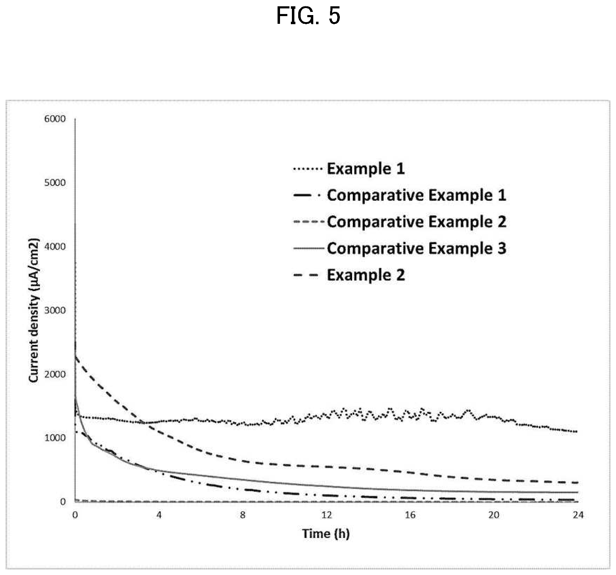

Electrochemical measurement was performed on the bioelectrodes produced in Examples 1 and 2 and Comparative Examples 1 to 3. The results are indicated in FIGS. 4 and 5.

Specifically, the measurement was performed in a stationary solution at 25°C in a three-electrode system using a silver-silver chloride electrode for a reference electrode, a platinum wire for a counter electrode, and a potentiostat. A solution obtained by adding glucose, to a concentration of 0.1 M, to 8 mL of a 0.1 M phosphate buffer was used as the solution.
Cyclic voltammetry conditions indicated in FIG. 4
Potential sweep range: from -0.2 V to 0.4 V (vs. silver-silver chloride electrode)
Potential sweep rate: 25 mV s⁻¹
Chronoamperometry condition indicated in FIG. 5
Applied potential: 0.3 V (vs. silver-silver chloride electrode)

As indicated in FIGS. 4 and 5, the bioelectrodes of the Examples, containing ZIF and CNT together with 12NQSO, exhibited superior magnitude of current density and durability of current density to the bioelectrodes of the Comparative Examples, not containing ZIF or CNT.

A bioelectrode was produced in the same manner as in Example 1 except that the concentration of the mediator was changed to 80 µM, and electrochemical measurement (chronoamperometry, applied potential: 0.3 V (vs. silver-silver chloride electrode), solution temperature: 25°C) was performed.

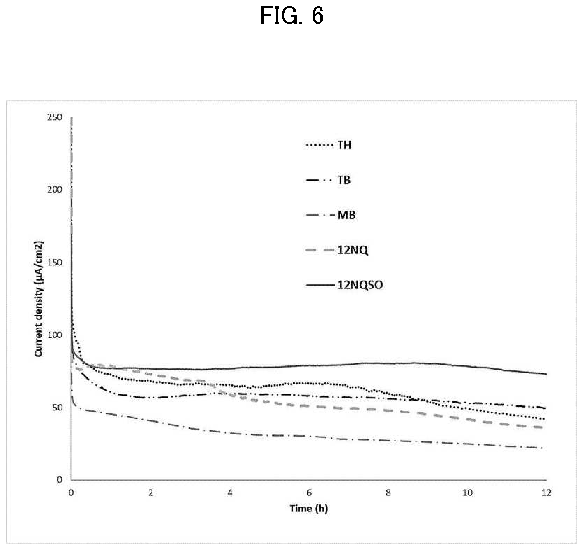

As the mediator, 12NQSO, 1,2-naphthoquinone (12NQ), thionine (TH), toluidine blue (TB), and methylene blue (MB) were used, respectively. The results are presented in FIG. 6.

As indicated in FIG. 6, the bioelectrode of Example 1 using the mediator having an anionic group (12NQSO) showed a higher current density than the bioelectrodes using the mediator having no anionic group (12NQ, TH, TB, MB).

### <Evaluation of concentration dependence of sensitivity to glucose>

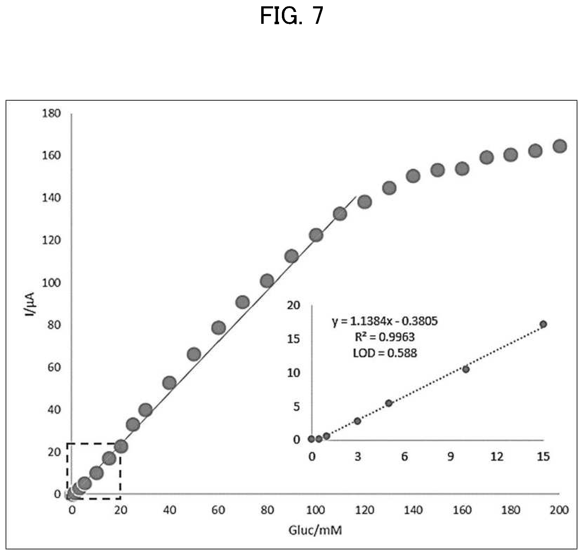

The bioelectrode produced in Example 1 was used to evaluate the sensitivity to glucose with different concentrations. The results are presented in FIG. 7.

Specifically, the electrochemical measurement (chronoamperometry, applied potential: 0.3 V (vs. silver-silver chloride electrode), solution temperature: 25°C) of the bioelectrode was performed according to the following procedure.

A glucose solution is added to 4 mL of a 0.1 M phosphate buffer so as to achieve a desired final concentration, and the mixture is stirred with a stirrer and allowed to stand until the stirring is stopped. After the stirring is stopped, electrochemical measurement is performed. A current value after 200 seconds from the start of the measurement is recorded. The above-described operation is repeated while changing the glucose concentration.

As indicated in FIG. 7, the bioelectrode produced in Example 1 exhibited excellent sensitivity to a wide concentration range of glucose. Since the blood sugar level of a healthy person is from 4.9 mM to 6.9 mM (blood) and from 3.9 mM to 6.6 mM (interstitial fluid), the bioelectrode produced in Example 1 is useful as an electrode of a blood sugar level measurement device.

### <Evaluation of selectivity of sensitivity to glucose>

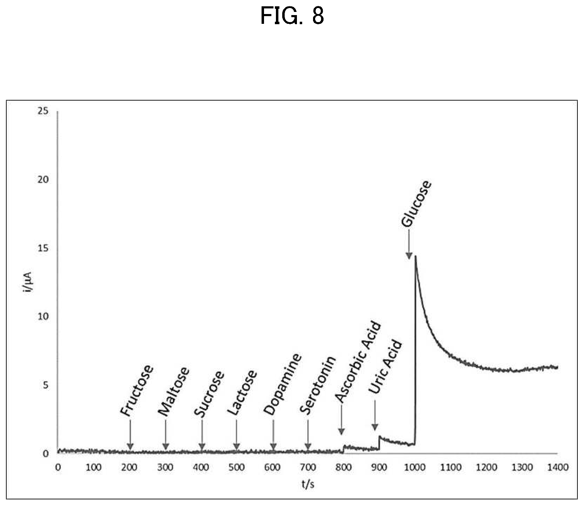

The bioelectrode produced in Example 1 was used to evaluate the selectivity to glucose. The results are presented in FIG. 8.

Specifically, the electrochemical measurement (chronoamperometry, applied potential: 0.3 V (vs. silver-silver chloride electrode), solution temperature: 25°C) of the bioelectrode was performed according to the following procedure.

To 4 mL of a 0.1 M phosphate buffer, fructose (5 mM), maltose (5 mM), sucrose (5 mM), lactose (5 mM), dopamine (0.1 mM), serotonin (1 mM), ascorbic acid (0.1 mM), uric acid (0.4 mM), and glucose (5 mM) are added in this order. After the addition of the substances, the solution is stirred with a stirrer, the measurement is started after the stirring is stopped, and the current value for 200 seconds from the start is recorded. The measurement is temporarily stopped from the addition of the substances to the start of the measurement.

As indicated in FIG. 8, the bioelectrode produced in Example 1 exhibited high selectivity to glucose.

### <Evaluation of durability of sensitivity to glucose>

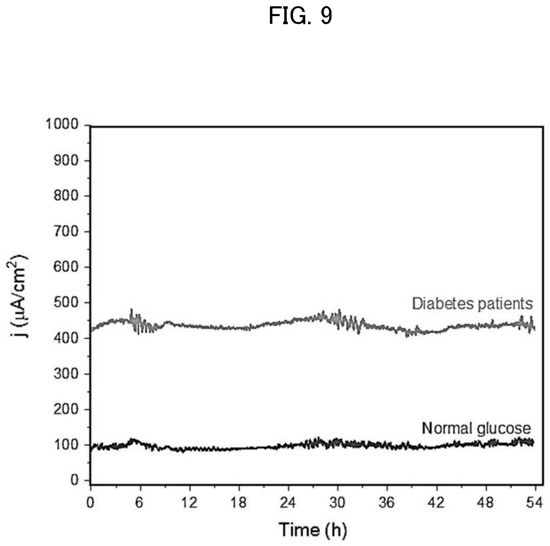

The bioelectrode produced in Example 1 was used to evaluate the durability of the selectivity to glucose. The results are presented in FIG. 9.

Specifically, the electrochemical measurement (chronoamperometry, applied potential: 0.3 V (vs. silver-silver chloride electrode), solution temperature: 25°C) of the bioelectrode was performed according to the following procedure.

In the measurement, a solution obtained by adding a glucose solution so as to achieve a desired final concentration of 20 mM or 5 mM to 4 mL of a 0.1 M phosphate buffer was used as the solution.

As indicated in FIG. 9, the bioelectrode produced in Example 1 maintained the sensitivity to glucose in the case with a glucose concentration of the blood sugar level of a diabetic patient (20 mM) and in the case with a glucose concentration of the blood sugar level of a healthy person (5 mM), even after 54 hours from the start of the measurement.

### <Example 3>

A bioelectrode was obtained in the same manner as in Example 1 except that a mixture of lactic acid oxidase (LOx), chitosan, and genipin was used in place of the mixture of FAD-GDH, chitosan, and genipin used for producing the bioelectrode in Example 1.

The mixture of LOx, chitosan, and genipin was prepared by mixing a solution containing 1 mass% of chitosan and 1 mass% of acetic acid (10 µL), a 25 mg/mL LOx solution (20 µL), and a solution obtained by dissolving 30 mg of genipin in 1 mL of ethanol (5 µL).

### <Evaluation of concentration dependence of sensitivity to lactic acid>

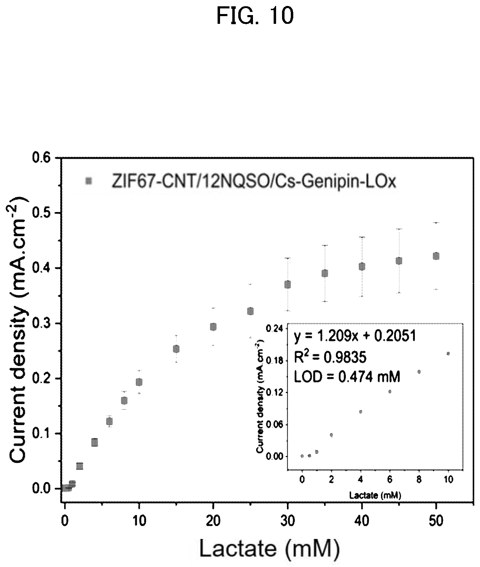

The bioelectrode produced in Example 3 was used to evaluate the sensitivity to lactic acid with different concentrations. The concentration dependence of the sensitivity to lactic acid was evaluated in the same manner as in the evaluation of the concentration dependence of the sensitivity to glucose described above. The results are presented in FIG. 10.

As indicated in FIG. 10, the bioelectrode produced in Example 3 exhibited excellent sensitivity to a wide concentration range of lactic acid.

### <Evaluation of durability of sensitivity to lactic acid>

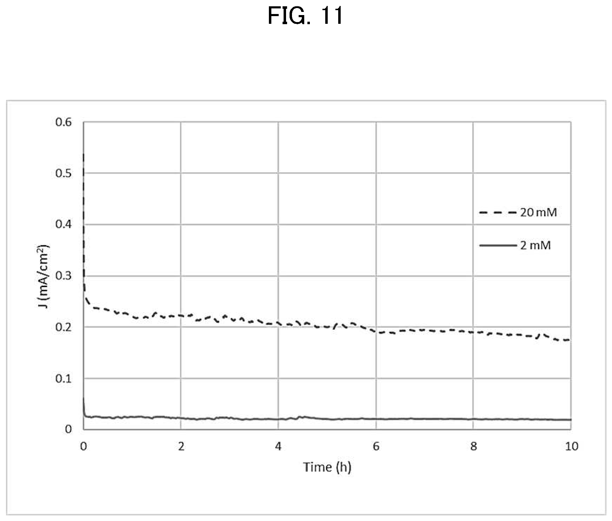

The bioelectrode produced in Example 3 was used to evaluate the durability of the sensitivity to lactic acid (2 mM or 20 mM). The results are presented in FIG. 11.

The durability of the sensitivity to lactic acid was evaluated in the same manner as in the evaluation of the durability of the sensitivity to glucose described above. The results are presented in FIG. 11.

As indicated in FIG. 11, the bioelectrode produced in Example 3 maintained the sensitivity to lactic acid even after 10 hours from the start of the measurement.

### The disclosure of Japanese Patent Application No. 2023-140572 is incorporated herein by reference in its entirety.

All the documents, patent applications, and technical standards described in the present description are hereby incorporated by reference to the same extent as in cases where each document, patent application, or technical standard is concretely and individually described to be incorporated by reference.

## Claims

1. A bioelectrode, comprising:
a complex compound composed of a metal and a ligand;
a mediator having an anionic functional group; and
a conductive material.

2. The bioelectrode according to claim 1, wherein the metal includes cobalt or zinc.

3. The bioelectrode according to claim 1, wherein the ligand includes an imidazole compound.

4. The bioelectrode according to claim 1, wherein the complex compound includes a metal organic framework.

5. The bioelectrode according to claim 1, further comprising a biocatalyst.

6. The bioelectrode according to claim 1, further comprising chitosan.

7. An electrochemical device, comprising the bioelectrode according to any one of claim 1 to claim 6.

8. A blood sugar level measurement device, comprising the bioelectrode according to any one of claim 1 to claim 6.

9. A concentration measurement method, comprising:
bringing the bioelectrode according to any one of claim 1 to claim 6 into contact with a liquid; and
measuring a concentration of a substance contained in the liquid.
